# EUROPEAN PATENT SPECIFICATION

(11) **EP 2 997 992 B1**
(45) Date of publication and mention of the grant of the patent: **23.06.2021**
(21) Application number: 14798288.8
(22) Date of filing: 09.05.2014
(51) Int. Cl.: A61M 25/04, A61F 2/966, A61F 2/95

(54) **MECHANISM FOR GUIDING AND/OR RELEASING AN ENDOPROSTHESIS AT A BLOOD VESSEL LESION REGION, USED IN A MEDICAL DEVICE OF THE TYPE OF A CATHETHER**
MECHANISMUS ZUM FÜHREN UND/ODER LÖSEN EINER ENDOPROTHESE IN EINER BLUTGEFÄSSVERLETZUNGSREGION IN EINER KATHETERARTIGEN MEDIZINISCHEN VORRICHTUNG
MÉCANISME DE CONDUCTION ET/OU DE LIBÉRATION D'UNE ENDOPROTHÈSE AU NIVEAU DE LA RÉGION LÉSÉE D'UN VAISSEAU SANGUIN, APPLIQUÉ À UN DISPOSITIF MÉDICAL DE TYPE CATHÉTER

(30) Priority: 13.05.2013 BR 102013011778; 05.05.2014 BR 132014010807
(43) Date of publication of application: 23.03.2016
(73) Proprietor: Inside Medical Indústria e Comércio de Produtos Médicos Hospitalares Ltda - ME, 88301-410 Itajaí - SC (BR); Gomes Nogueira, Ana Paula, 88040-460 Florianópolis - SC (BR); Barbosa Mandelli, Marcelo, 88048-300 Florianópolis - SC (BR); Reis, Flávio, 88230-000 Canelinha - SC (BR); Mello Martins, Waldemar Fernando, 88303-101 Itajaí - SC (BR)
(72) Inventor: GOMES NOGUEIRA, Ana Paula, 88040-460 Florianópolis - SC (BR); BARBOSA MANDELLI, Marcelo, 88048-300 Florianópolis - SC (BR); REIS, Flávio, 88230-000 Canelinha - SC (BR); MELLO MARTINS, Waldemar Fernando, 88303-101 Itajaí - SC (BR)
(74) Representative: Delorme, Nicolas
(86) International application number: PCT/BR2014/000147
(87) International publication number: WO 2014/183178

(56) References cited:
- EP-A1- 1 205 208
- EP-A1- 2 289 408
- WO-A1-98/24501
- WO-A1-03/101518
- WO-A1-2006/037086
- WO-A1-2009/148594
- WO-A1-2011/081997
- WO-A2-2008/098255
- US-A1- 2006 095 119
- US-A1- 2009 048 656
- None

## Description

### FIELD OF APPLICATION

The present patent of invention with the title in compound and object of description and claims in this text relates to an inventive solution in the application field / scope appointed by the medical area, meeting particular utility concerning to treatment of vascular diseases, namely for specific pathologies such as aneurysms and aorta dissections.

### INVENTION DEMAND

The invention is as set forth in the appended claims. The development of the "mechanism for fastening and / or releasing an endoprosthesis with an injured region of a blood vessel applied in catheter-like medical devices" was motivated by urgent needs in make endoprosthesis implanting safer and faster when in surgical intervention, namely known surgical procedures such as catheterism, minimizing the introduction impact of a foreign body within the blood vessel, especially in throughout its path, since the penetration through an intake by a peripheral vessel up to the point of this vessel wherein the endoprosthesis will be indeed implanted.

From the perspective of the patient to be minimized the impact of the endoprosthesis passage along the blood vessel is added to guarantee that this medical procedure even though being minimally invasive will not injury the inner walls of the vessel, wherein once implanted the endoprosthesis, this will be perfectly functional, i.e. including quality assurance, ensuring an adequate blood flow in the vessel segment requiring intervention, in the sense that the patient passes to count on postoperative period provided with comfort on his heart activity.

### PARADIGM OF THE INVENTION

Inventor elects as development paradigm the current state of the art in admittedly effective surgical procedures in expandable endoprosthesis implanting, notably the well-known solution in medical area as "catheterism", characterized by involving the use of catheterism medical devices.

### REQUIREMENTS OF THE INVENTION

In accordance with the demand of the invention as well as the paradigm development of the invention, the inventor has devised a "mechanism for guiding and or releasing an endoprosthesis with an injured region of a blood vessel applied in catheter-like medical devices" provided with novelty, as it ensures the consolidation of a wealthy and proper blood flow in the vessel (artery segment, carotid arteries, iliac coronaries, for example), after their implant, minimizing the impact of vascular endoprosthesis passage through the inside of this vessel, i.e. ensuring the integrity of the wall segment still healthy, thanks to the reduced volume of the set "retracted endoprosthesis + transport mechanism, handle and so releasing the endoprosthesis".

Inventor points out that the unprecedented mechanism for transporting, handling, and releasing the endoprosthesis does not arise from obvious or evident way to other known solutions known to the state of the state of the art for such structures, and its constructive concept and corresponding functional concept are the fruit of effort in understanding subjects in the medical field, notably the anatomy of blood vessels associated with knowledge of implant procedures and techniques of catheter-like devices.

Further the "invention" comprises industrial applicability, being economically viable and, therefore, complying with the stringency of the requirements for patentability, notably as a patent of invention.

### TECHNICAL BACKGROUND

In order to provide truth to the explicit context in the introductory framework, it will be presented a brief explanation about the state of the art for expandable endoprosthesis such as "stent graft" as well as the techniques and devices used for their implementation, wherein it will be possible for a skilled in the art technician to recognize both its positive as limiting aspects, to later discuss the advantages aggregated with the introduction of unprecedented "mechanism for repositioning and or entire removing of an endoprosthesis next to an injured area of a blood vessel" claiming object in herein.
a. The expandable endoprosthesis device: also known technically the term "stent graft", it has revolutionized interventional practice for treating heart ischemic and symptomatic diseases. In clinical practice, they are being increasingly prescribed to a larger number of patients.
b. Technological basis in endoprosthesis: an expandable endoprosthesis for treatment of vascular diseases presents constructive concept formed by a metallic tubular structure, which can be cylindrical, conical or bipartite, and that can be presented in the form of a screen or mesh which wires are made of metallic material with a high degree of flexibility, allowing the expandable endoprosthesis to present small volume in relation to its implantation, by means of special catheter, and expansive volume after implanting it. In addition, said structure may receive a polymeric coating. In the current state of the art there are endoprosthesis structures formed by independent sections or by a mesh of metal wires, mainly steel and nitinol, which are sutured to the coating.
c. Endoprosthesis implantation technique: using "catheter-like" devices is conventional, wherein said ones can be defined as medical devices having as objective to promote the implant of an endoprosthesis in an injured region of a blood vessel, especially the aorta. Also known as "endovascular surgery", the procedure is performed by means of an incision, usually in the groin, where the catheter housing an endoprosthesis in its interior is introduced, being guided through a guide to the location defined by a specialist, in order to discover and release the endoprosthesis in the vessel segment to be treated, then being removed from the interior by the same route of entry.
   The use of endoprosthesis implant by means of catheterization is a minimally invasive procedure, being an option to open surgery in the treatment of injuries in the blood vessels.
d. Catheter device characterization: several models of catheter are known with the same main function of guiding an endoprosthesis through the vessels and release it in a specific location, wherein the critical observation of these solutions reveals that its constructive concepts are distinguished primarily by its constructive configuration, i. e., "design".
   Different operating mechanisms provide the catheters from various functions such as uncovering and releasing the endoprosthesis at the same time, uncovering with later releasing, and repositioning, entire endoprosthesis removal after partial clock, among others. In the endovascular surgical procedure the physician should guide the catheter to the location considered ideal for implanting the endoprosthesis. If the physician deems that the location is not the most appropriate during the uncovering and releasing of same, the endoprosthesis repositioning is necessary. Catheters having such a function are advantageous over others. In this sense there are catheters that besides repositioning have the possibility of endoprosthesis removal. In these cases, even after the endoprosthesis has been expanded (but not yet fully released); the doctor manages to imprison it again within the catheter, cover the sheath again and remove it from the patient. This generates even a greater safety to the patient.
   Catheter-like device solutions allowing both for repositioning as the entire endoprosthesis removal from the interior of the vessel after a "partial clock" of its expansion and releasing mechanism is found in the state of the art, wherein critical analysis of the same reveals that the endoprosthesis removal occurs by using a device in the form of a metallic wire basket, being necessary for the basket to be in the interior of the sheath and wraps the endoprosthesis in order to promote its imprisonment and subsequent removal.
e. Identification of the macro problem: although the use of a device in the form of a wire basket meets the objective of allowing to carry a compressed endoprosthesis with any catheter model, inventor identifies that the same features as technical aspect negative, the concrete fact that wire basket attached to the endoprosthesis, converges to a significant volume increasing in the volume of material on the interior of the sheath , thus demanding the specification of larger gauges catheters devices, and consequently presenting enough volume to generate any kind of problem during or after the implant procedure.

It is for example known from document WO 2011081997 A1 a delivery device for proximally releasing an expandable prosthesis and method of use thereof, used, for instance, in endoprostheses delivery systems for the colon and esophagus. Said device comprises an introducer sheath that is capable of distally advancing relative to the endoprosthesis to thereby expose the endoprosthesis from a proximal end thereof. The device is also capable of resheathing over the prosthesis in a proximal direction. This document describes the use of stabilizing elements which allow the prosthesis to keep engaged, even when the sheath is removed. Among said stabilizing elements, one can cite a tube with a loop. Said arrangement allows a wire to pass between the loop and the stent, holding them together. Said wire is, then, tied to a wire guide. When said wire guide is pulled, the wire is also pulled and untied, releasing the stent and allowing it to be pulled.

### PROPOSAL OF THE INVENTION

a. Invention paradigm development: according to the as evidenced in the topic of background of technique, the inventor has adopted as a paradigm for the development of the invention the technical rule on which it is set the volume of material contained within a catheter is what determines its diameter and, the smaller volume, the easier the doctor accesses the location of the implant, and consequently the trauma caused to the patient is lower.
b. Objective of the invention: considering the "catheter" device and the paradigm of development of the invention, the inventor converged in a solution such that in addition to preserve the conventional functions embedded in it, aims to enable the repositioning of an endoprosthesis or the removal of the same from a simple system that significantly reduces the volume of material in the interior of this catheter.
c. Constructive concept of the invention: in the following text, and until the end of this, a reference system is used to describe the ends of the catheter. It is said "proximal" the region of the catheter that during a procedure is closer to the heart. While the expression "distal" makes reference to the opposite end.

For the solution proposed herein, the endoprosthesis is compressed inside the catheter and fastened to the same in its proximal and distal regions, wherein the fastening system is responsible for the final release of the endoprosthesis and the fastening system of the distal region is responsible for repositioning and removing the endoprosthesis.

After accessing the location of the implant with the catheter, the doctor uncovers the sheath and the endoprosthesis entrapped therein expands due to its self-expansion property. However the same keeps attached to the catheter in its proximal and distal regions.

The system that provides the possibility of repositioning and removing the endoprosthesis to the catheter consists of a tube which contains in its proximal region a wire that attaches the distal region of the endoprosthesis. This tube is connected to a button on the distal region of the catheter thereby allowing its movement. If after the endoprosthesis has already expanded, the doctor moves this button in the distal direction, the tube that fastens the distal part of the endoprosthesis is moved and pulls the same, thus reducing its diameter enabling the endoprosthesis to be covered by the sheath. In this way, the catheter is closed again, with the endoprosthesis entrapped inside it and it is possible to remove said endoprosthesis.

The system components of repositioning and removing the endoprosthesis are arranged in the catheter so as to occupy only the spaces empty before, thus not adding volume to the interior of the catheter wherein the endoprosthesis is housed. The tube responsible for the movement of the endoprosthesis is distally allocated in relation to the same, said being in contact only by the wire that connects them.

### DESCRIPTION OF THE DRAWINGS

Complementing this description to provide a complement to the description of the specification in order to gain a better understanding of the characteristics of the present invention patent, a set of drawings is attached in appendices, that renders a preferred embodiment for the "mechanism for guiding and or releasing an endoprosthesis with the injured region of a blood vessel, applied in catheter-like medical devices, wherein:
Figure 1 shows a perspective view of a catheter-like device;
Figure 2 is an exploded view in perspective of constructive arranging a catheter-like device;
Figure 3 shows a catheter-like device with the sheath shifted in the distal direction;
Figure 4 shows a catheter-like device with an uncovered and pulled endoprosthesis;
Figure 5 shows in detail a pulled endoprosthesis applied to a catheter-like device;
Figure 6 shows in detail the distal fastening of the endoprosthesis with a catheter-like device;
Figure 7 shows in detail the distal fastening wire attached to the tether.
Figure 8 shows in detail the released distal fastening wire;
Figure 9 shows a cutaway view of the capillary tube and aggregate components embedded in a catheter-like device;
Figure 10 shows a cutaway view of the movable multiway tube on the capillary tube, among other components applied in a catheter-like device;
Figure 11 shows a cutaway view of the reinforcing tube on the movable multiway tube, among other components applied in a catheter-like device;
Figure 12 shows a cutaway view of the tether and tether button, among other components applied in a catheter-like device;
Figure 13 shows the sheath over the reinforcing tube among other components applied in a catheter-like device;
Figure 14 shows a schematic of an artery with a guidewire introduced;
Figure 15 shows a schematic of an artery with a catheter device being introduced;
Figure 16 shows a schematic of an artery with a catheter-like device introduced, provided with an uncovered endoprosthesis;
Figure 17 shows a schematic of an artery with an introduced catheter-like device with an expanded endoprosthesis in its interior;
Figure 18 shows the schema of an artery with a catheter-like device provided with an endoprosthesis having its distal portion released; and
Figure 19 shows a schematic of an artery with a catheter-like device provided with a fully released endoprosthesis;
Figure 20 shows a perspective view of the invention, showing the dimensional reduction of the component parts;
Figure 21 shows an enlarged perspective view of the invention showing the fastening mechanism of the endoprosthesis distal portion;
Figure 22 shows an expanded perspective view of the invention showing the releasing mechanism of the endoprosthesis distal portion;
Figure 23 is an illustrative representation of a third embodiment of the component piece of reinforcing tube;
Figure 24 is an illustrative representation of a third embodiment of the component piece of multiway tube;
Figure 25 is an illustrative representation of a fourth embodiment of the component piece of reinforcing tube;
Figure 26 is an illustrative representation of a fourth embodiment of the component piece of multiway tube;
Figure 27 shows a rear perspective view and in cross-section from the distal portion of the second embodiment of the catheter; and
Figure 28 is an illustrative representation of proximal fastening elements of the endoprosthesis for the second embodiment of the catheter.

### DETAILED DESCRIPTION

The following detailed description should be read and interpreted with reference to the drawings presented, representing two embodiments for the "mechanism for driving and or releasing an endoprosthesis with an injured region of a blood vessel, applied in catheter-like medical devices", as well as its obtaining process, not intended to limit the scope of the invention, said being limited only to the stated in the set of claims.

### A. First embodiment.

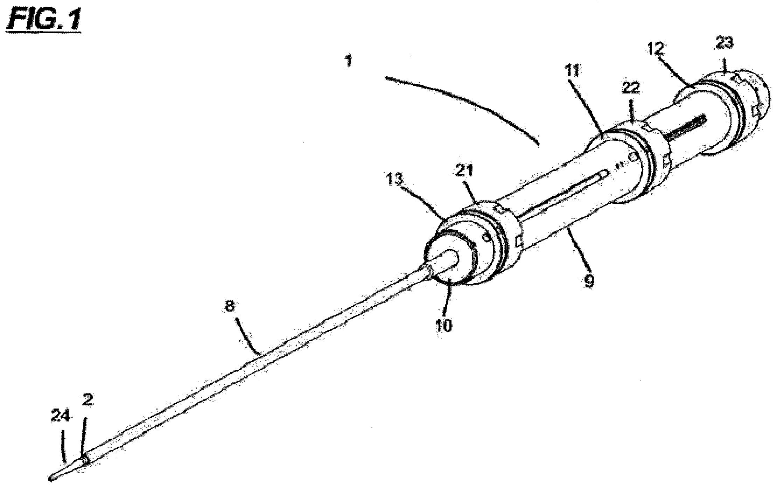

A1. Constructive concept: as evidenced in Figure 1 showing the catheter (1) in the completely closed position, being disclosed the following components: a tip (24) being on its proximal region, wherein said tip (24) is responsible for the punching of vessels, such as the iliac artery, for example, for the introduction of said catheter (1), the tip base (2), being a radiopaque element which serves as a reference during the procedure. It is also defined a sheath (8) with the aim of housing an endoprosthesis in its interior; the proximal lid (10), sheath movement button (13) and the sheath (8), the sheath button lock (21) that only when rotated allows the offset of the sheath, the grip (9) which is the support of all the catheter commands, the reinforcing tube button (11), the reinforcing tube button lock (22) which does not allow for the displacement of the reinforcing tube (7) without being rotated the movable multiway tube (12) and the movable multiway tube lock (23) having similar function to the other locks.

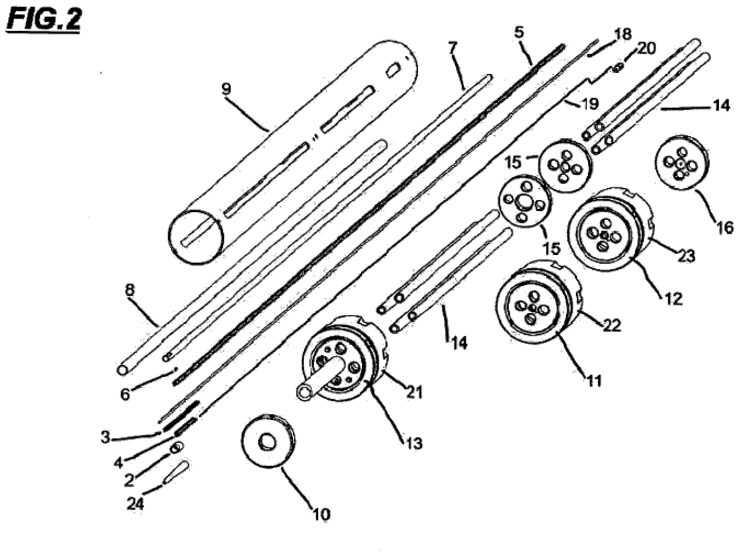

In Figure 2 is shown an exploded view of the catheter (1) where can be seen the tip (24), tip base
(2), fix multiway tube (4) tube, capillary tube (18), sheath (8), proximal lid (10), four proximal fastening pins
(3), movable multiway tube (5), movable multiway tube button (12), rods (14), distal fastening wire (6), reinforcing tube (7), reinforcing tube button (11), sheath button lock (21), button lock of the reinforcing tube (22), button lock of the movable multiway tube (23), tether(19), tether button (20), distal lid (16), grip (9), sheath movement button (13) and the intermediate lids (15).

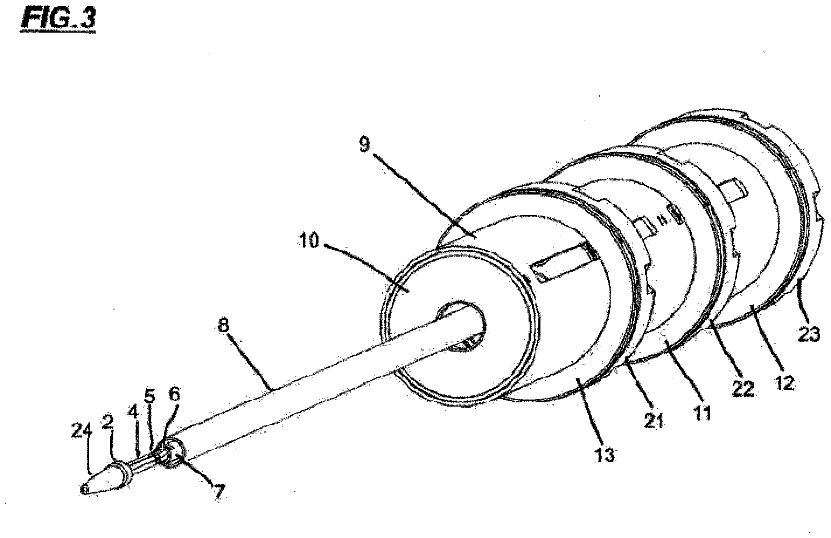

Figure 3 shows the catheter (1) without an endoprosthesis, with the sheath (8) shifted to the distal region, allowing the visualization of internal components to the sheath as, for example, the tip base (2), the fix multiway tube (4), the movable multiway tube (5), the distal fastening wire (6), and reinforcing tube (7).

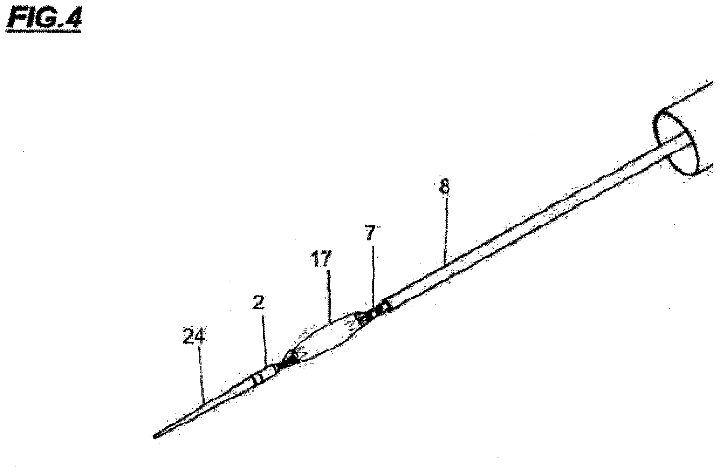

Figure 4 shows the catheter (1) with an endoprosthesis (17). The endoprosthesis is pulled by the reinforcing tube (7).

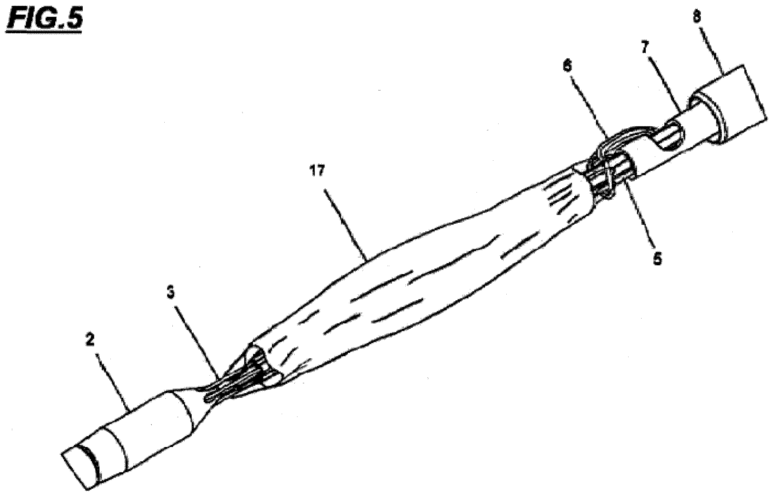

Figure 5 is shows in detail the fastening of the endoprosthesis (17) into the reinforcing tube (7) by means of the distal fastening wire (6), the proximal fastening pins (3), which are affixed to the movable multiway tube (5), pass through the inside of the movable multiway tube (4) and finally fit on the tip base (2), the movable multiway tube (5) when shifted in the distal direction causes the proximal fastening pins (3), affixed to the end of the movable multiway tube, disengage from the tip base (2) and of the endoprosthesis (17), releasing the proximal region of the same.

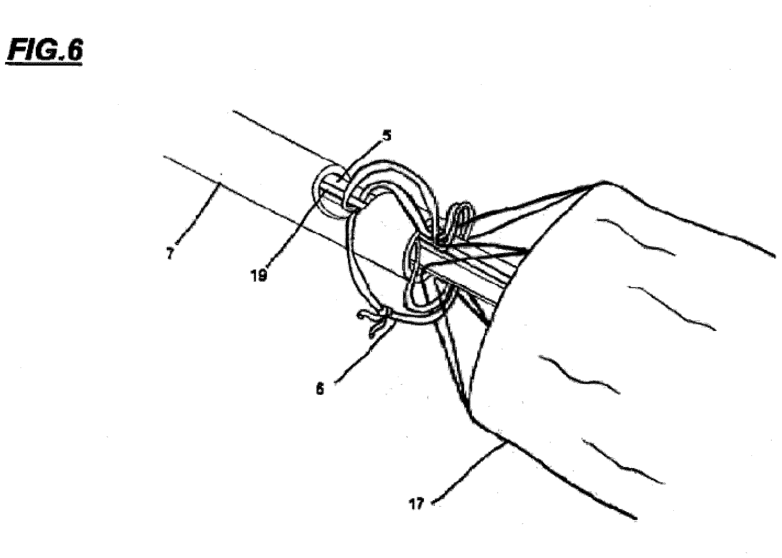

In Figure 6 is shown in detail the fastening of the distal region of the endoprosthesis (17). The prosthesis is attached to the reinforcing tube (7) through the distal fastening wire (6), said one being secured by its ends to the reinforcing tube (7) and tether (19), interlocking the endoprosthesis (17).

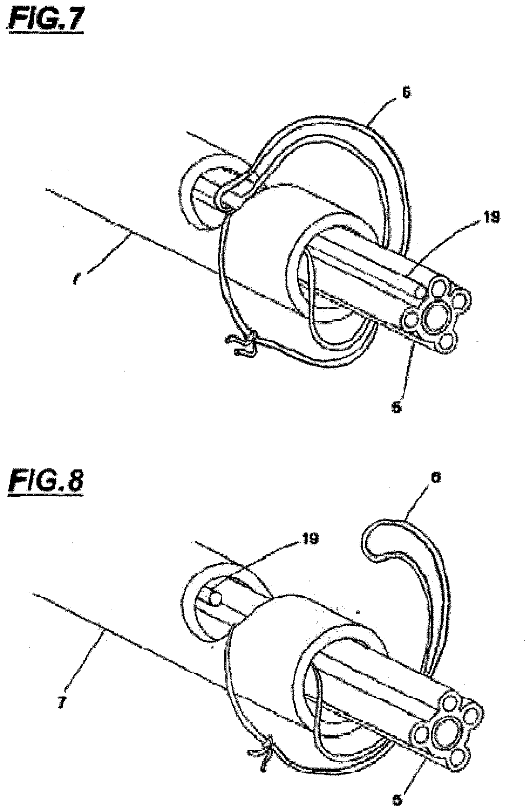

In Figure 7 is shown in detail distal fastening wire (6) with one end secured to the reinforcing tube and the other, secured between the tether (19) and the movable multiway tube (5). The distal fastening wire (6) has one of its ends released as the tether (19) is shifted, so its end trespasses the reinforcing tube hole (7).

In Figure 8 it is possible to observe the tether (19), shifted in the direction of the distal portion of the catheter, between the reinforcing tube (7) and movable multiway tube (5), allowing releasing the distal fastening wire (6) and subsequent releasing the endoprosthesis.

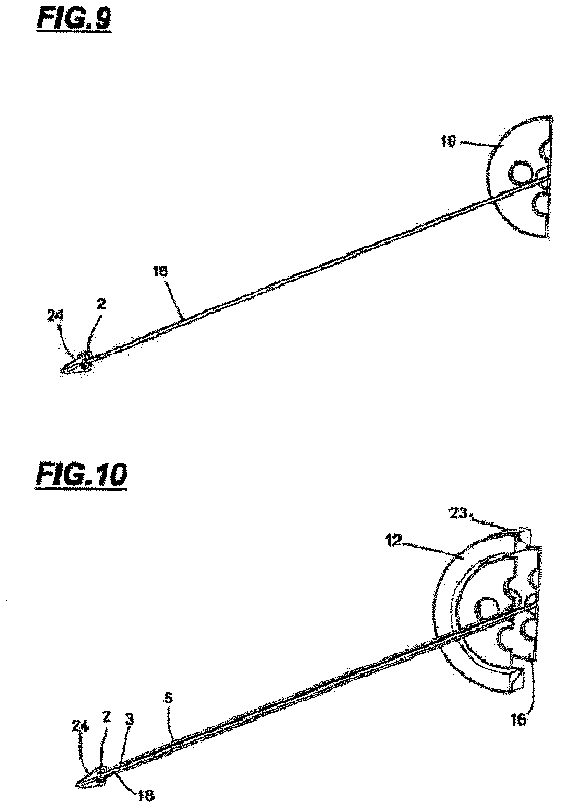

Figure 9 shows a cutaway view of portions of the catheter where it is possible to observe the capillary tube (18) which has the distal lid (16) fastened to its distal end and, in its proximal end the tip (24) and the tip base (2).

Figure 10 shows a cutaway view of portions of the catheter wherein it is possible to observe the multiway tube (5) which has affixed in its distal end the multiway tube button (12) and, in its proximal end, the fastening pins (3) fitting in the tip base (2). Multiway tube (5) moves freely over the capillary tube (18) on its longitudinal axis, under its button command.

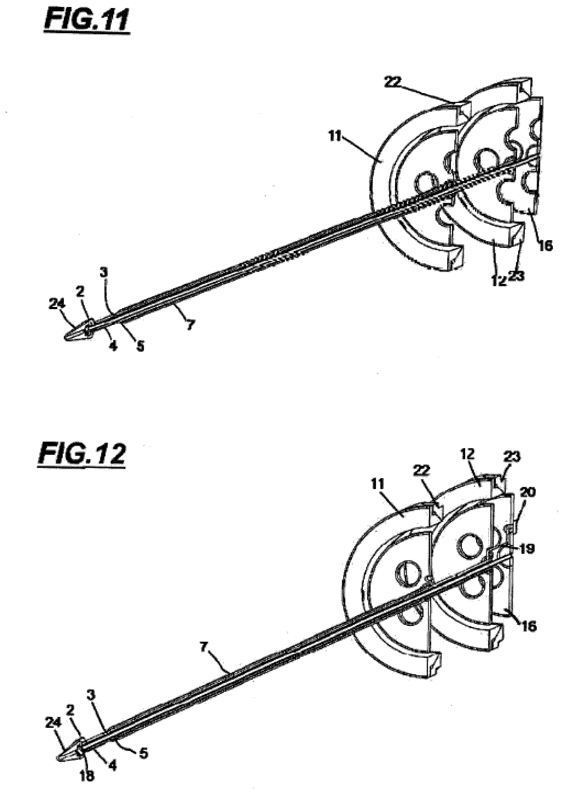

Figure 11 shows a cutaway view of parts of the catheter wherein it is possible to observe the reinforcing tube (7) which has fastened in its distal end the button of the reinforcing tube (11). Reinforcing tube (7) moves freely under its button command, in its longitudinal axis, over the multiway tube (5).

Figure 12 shows a cutaway view of parts of the catheter where it is possible to observe the tether (19) the button tether (20) which is fitted in distal lid (16), and from this lid, the tether (19) extends along the catheter until it gets next to the fix multiway tube (4), this being affixed into the capillary tube (18).

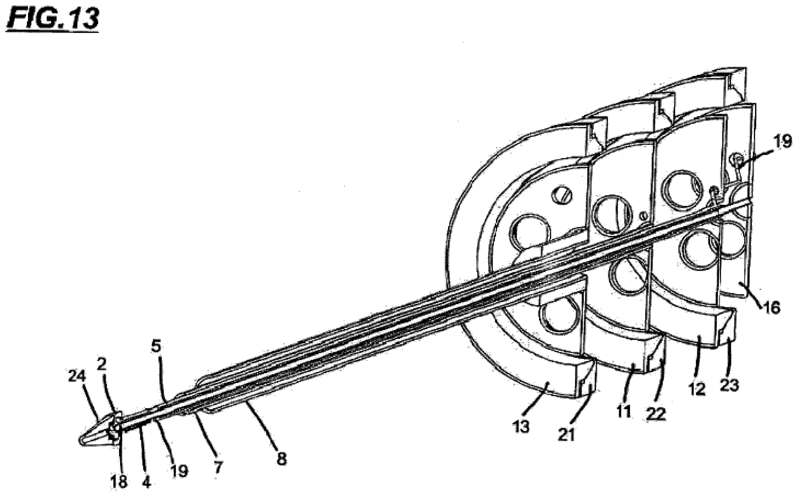

Figure 13 shows a cutaway view of portions of the catheter positioned so that it is possible to fully observe the tether (19) which extends along the catheter from the distal lid (16), passing through a gap between the movable multiway tube (5) and reinforcing tube (7) until gets next to the movable multiway tube (4).

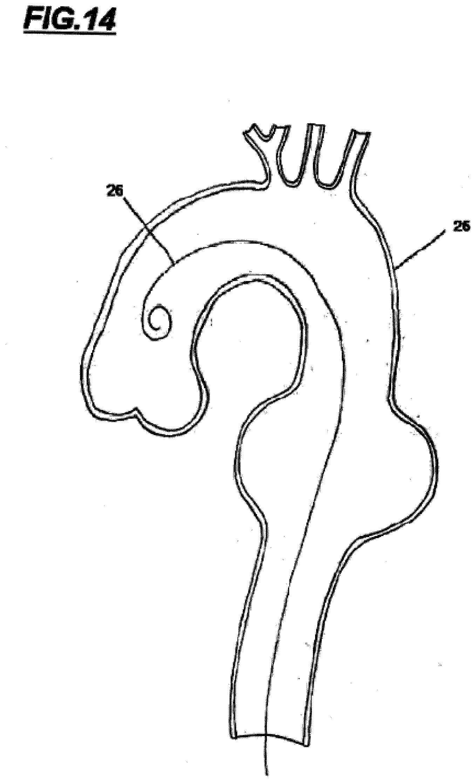

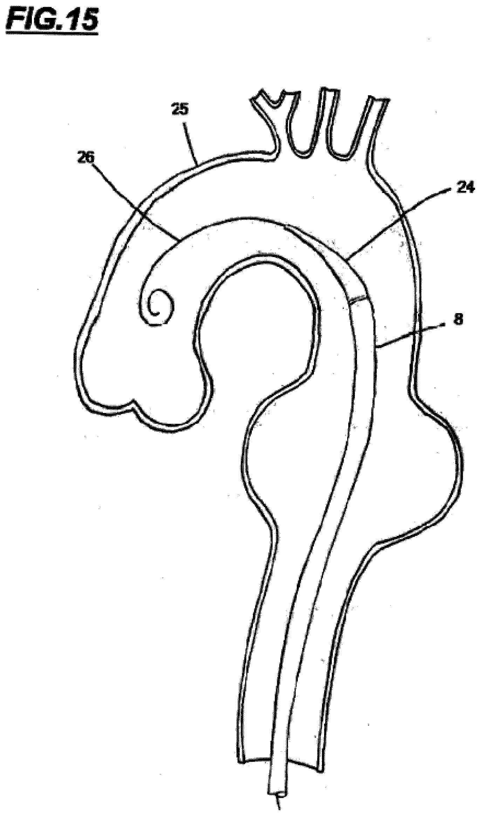

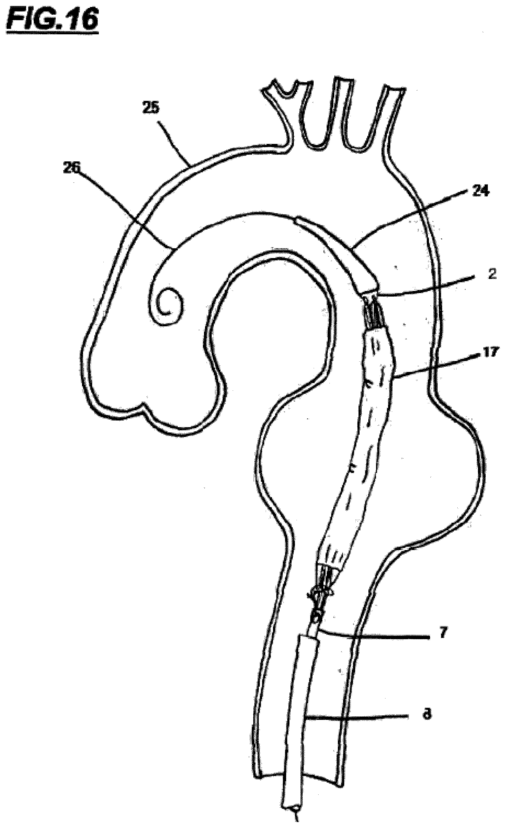

A2. Operational concept: as evidenced by the figures 14 and 15, the catheter (1) first advances over a guidewire already introduced in the artery to the location defined by a specialist using the base of the tip (2), which is a radiopaque element. When in the defined location, the button lock of the sheath (21) is removed, the grip (9) is kept stationary and the sheath movement button (13) is shifted in the distal direction until the endoprosthesis (17) is uncovered completely by the sheath (8), as can be seen in Figure 16.

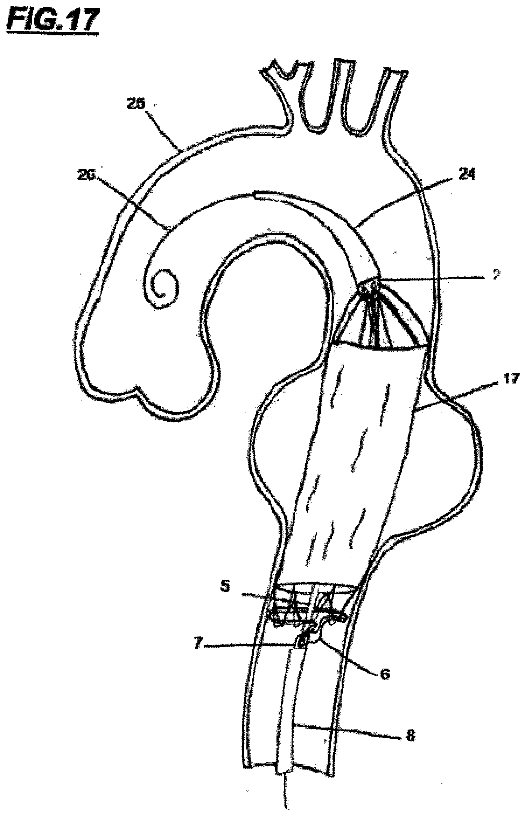

By means of button lock releasing of the reinforcing tube (22) and the movement of the reinforcing tube button (7) on the proximal direction, the endoprosthesis (17) is expanded and positioned as shown in Figure 17. If necessary an endoprosthesis (17) repositioning, the reinforcing tube (22) is shifted in the distal direction, with the endoprosthesis (17) and consequently reducing its diameter, as can be seen in Figure 16, so allowing its repositioning and later expansion.

If it is necessary to remove the endoprosthesis (17), reinforcing tube button (22) to its original position, so that the endoprosthesis (17) be pulled, as shown in Figure 16, for then the sheath movement button (13) to be shifted in the proximal direction until it returns to its original position, with the grip (9) must be kept stationary. With this the endoprosthesis (17) is covered again by the sheath (8) as shown in Figure 15, and the catheter (1) can be removed from the patient.

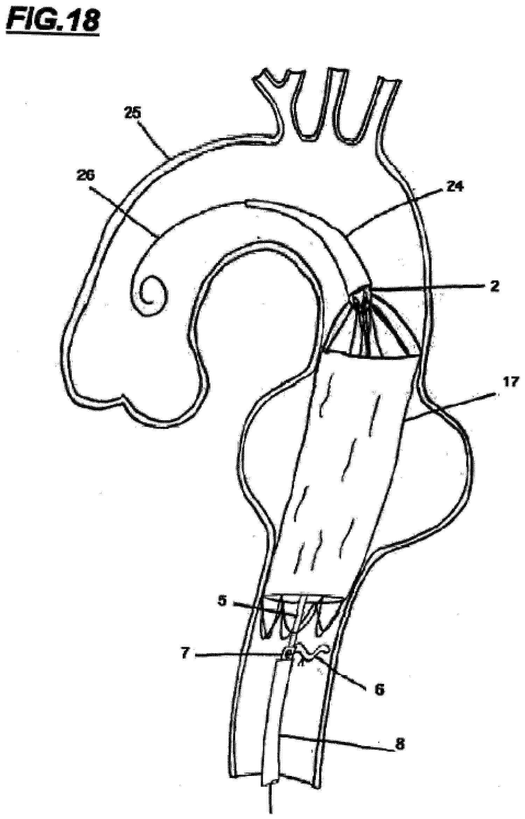

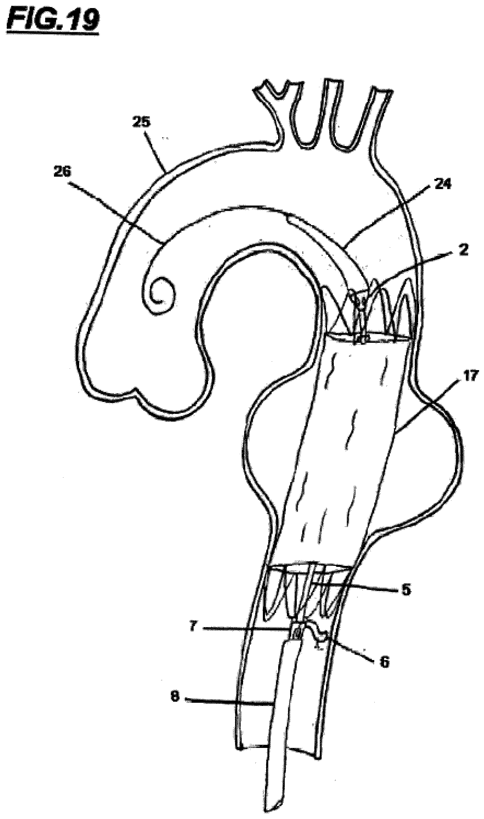

It is valid to notice that for covering again the endoprosthesis (17), the same should meet its distal and proximal portion fastened to the catheter (1). When placed in the correct location, the distal region of the endoprosthesis (17) can be released, as can be seen in Figure 18, and for that it is necessary the removal or just the indentation of the tether (19), by pulling the tether button (20). Thereafter the distal fastening wire (6) is released and can be removed from the endoprosthesis (17) shifting the reinforcing tube button (11) in the distal direction. Endoprosthesis (17) can be fully released, as can be seen in Figure 19, through the removal of the lock of the movable multiway tube (23) and the shifting of the multiway button (12) on the distal direction, culminating in the detaching of the proximal fastening pins (3) from the tip base (2).

B. This embodiment of the invention as set forth in the appended claims in synthesis is a notorious improvement importance in relation to the first embodiment already disclosed, notably giving enhances in constructive point of view itself without, however, giving up all the benefits listed exhaustively in the proposal topic of the invention.

In this manner, with as paradigm the first embodiment disclosed, the improvements were introduced along with its mechanism for driving and or releasing endoprosthesis in the injured region of a blood vessel, which expected technical effect resides in allowing repositioning of this endoprosthesis and or its removal from a simple system that significantly reduces the volume of material in the interior of this catheter.

From industrial point of view, this improvement developed reflects the explicit need to introduce a catheter manufacturing process as a whole provided with differentiated productivity and consequent reduction of its industrial cost, which therefore reflects in the profit margins "mark up" for marketing said new catheter wherein it is possible to affirm that finally gives the copyright owner of this new technology a competitive edge.

Finally yet in relation to the first form embodiment it has been identified an emerging need to impart the catheter for endoprosthesis application greater operational efficiency, i.e. higher quality and ergonomics when the catheter is taken to an operational condition.

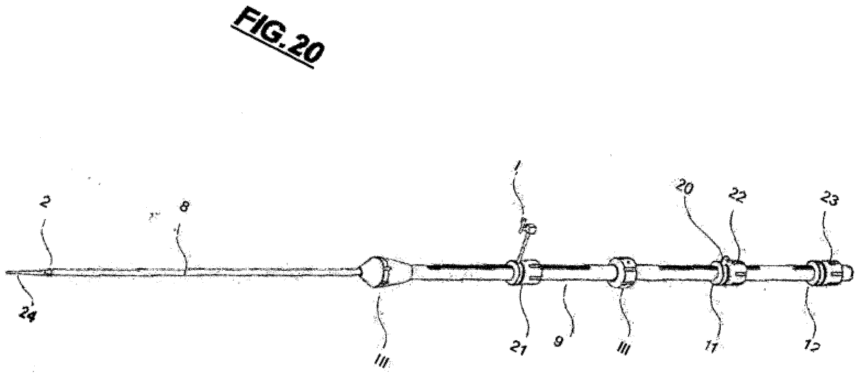

B1 Catheter General Constructive Concept: as evidenced in Figure 20, this presents a reduction in the diameter of the handle (23), as well as are eliminated structural rods set in the originally filed embodiment, the location of lifter button (20) is changed and included the washing valve components (I), proximal gripper (II) and medium gripper (III), and it is still performed the reduction in diameter of buttons (21) and (22).

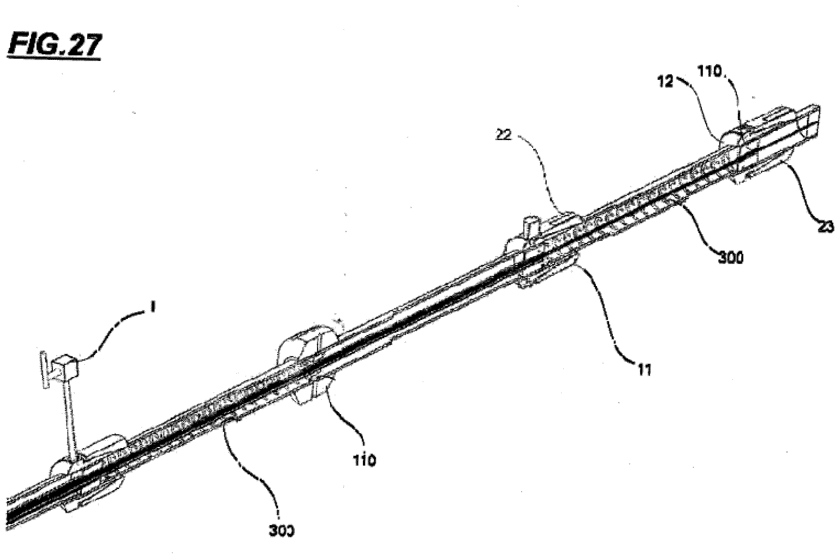

In turn, as evidenced in Figure 27 reinforcing ring (110) and springs (300) were inserted to assist in the structure mechanical resistance.

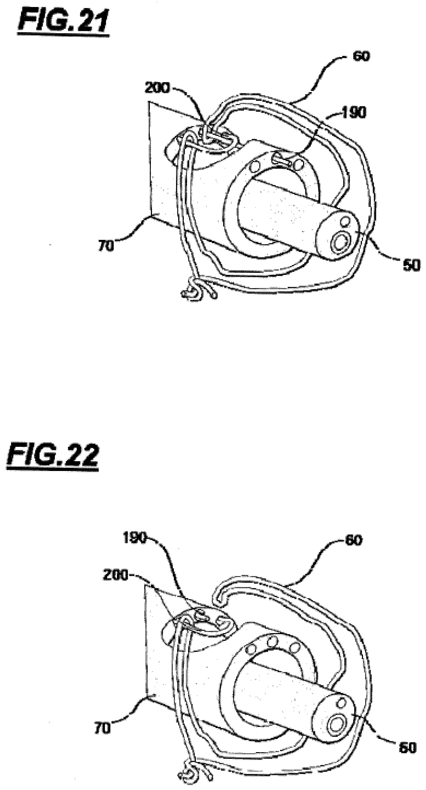

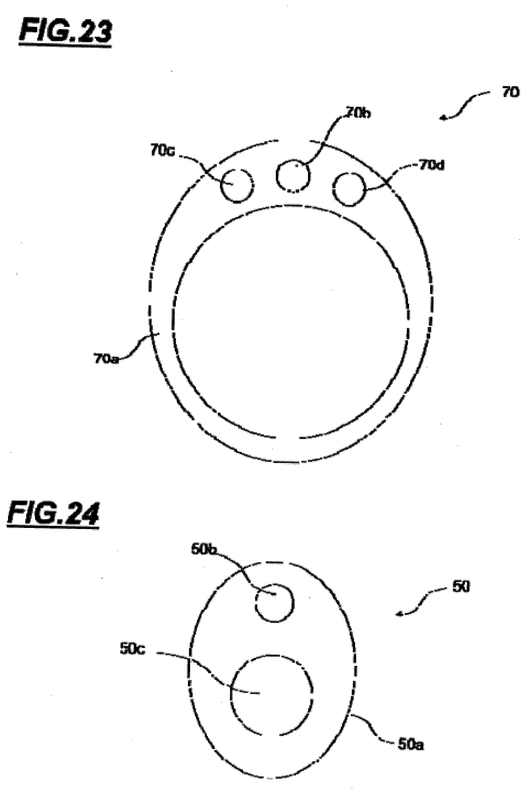

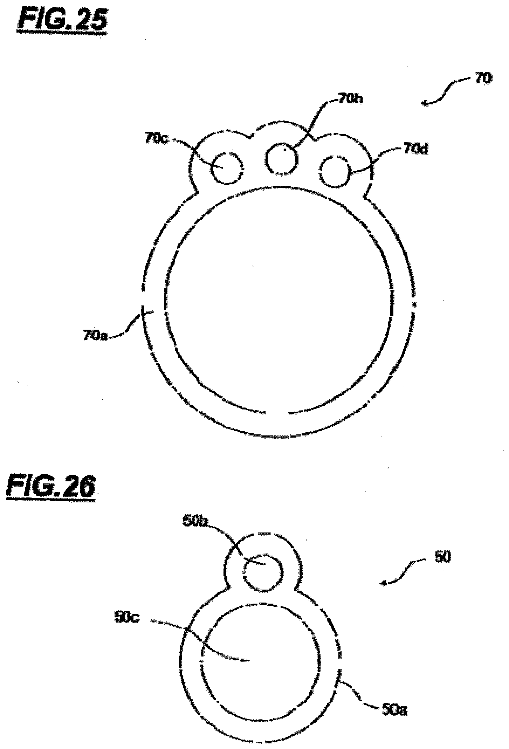

B2. Concept of fastening and releasing of the endoprosthesis distal portion: as evidenced in figures 21, 24 and 26 respectively, the fix multiway tube (50) that was movable and had 5 ways now is fastened and comprises only 2 channels (50a and 50b).

In turn, as shown in figures 21, 23 and 25 respectively, the reinforcing tube (70) that had only 1 way, now has 4 ways (70a), (70b), (70 c) and (70 d) respectively.

The fastening of the distal portion of the endoprosthesis is made by the same distal fastening wire (60) that in this new embodiment is secured to the tether (190) and still by a wire (200) that passes through two ways (70 c) and (70) of the reinforcing tube (70);

In turn this wire (200) extends to the button (11), sees Figure 27 of reinforcing tube (70) which being moved moves said reinforcing tube of which technical effect is to provide the endoprosthesis expansion and contraction.

Tether (190) passes through one of the reinforcing tube (22) ways. In addition, the button, the tether (20) is positioned on the button of said reinforcing tube (11).

The releasing of the endoprosthesis distal with the tether (190) being shifted in the direction of the distal part of the catheter, allowing the releasing of distal fastening wire (60) and consequently releasing the endoprosthesis distal portion as evidenced in Figure 22.

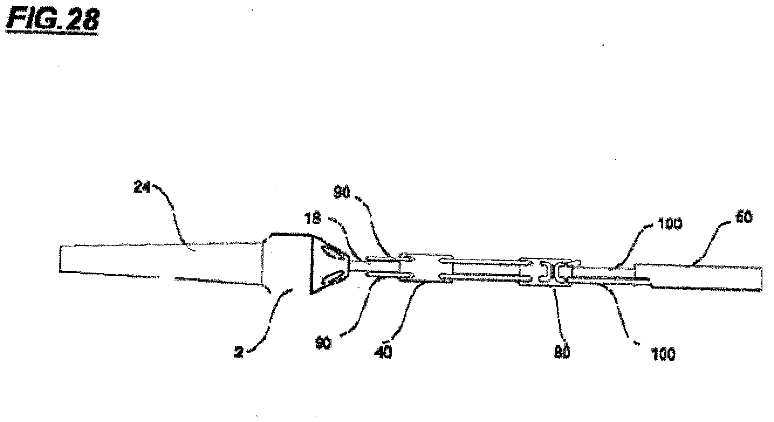

B3. The concept of proximal fastening and releasing the endoprosthesis: the elements that promote the fastening and releasing of the endoprosthesis proximal portion is a five-way fix multiway tube (40), four proximal fastening pins (90), one two-way fix multiway tube (50), as evidenced in Figure 28.

In this new embodiment the fix multiway tube (50) keeps fixed (in the version originally filed the multiway tube (5) moves).

In turn the ways of the two-way fix multiway tube (50), there is a wire (100) passing which will be connected to the button (12) on the catheter handle, see Figure 20. This wire (100) will be connected at the other end to a small tube (80) with five ways, wherein 4 of said ways will be fastened on the four proximal fixation pins (90) as shown in Figure 28.

For carrying out the release of the endoprosthesis proximal part, the button (12) is moved, said button moves the wire (100) and said wire moves the small tube (80) accordingly moving the four proximal fastening pins (90).

## Claims

1. MECHANISM FOR GUIDING AND/OR RELEASING AN ENDOPROSTHESIS WITH INJURED REGIONS OF A BLOOD VESSEL, APPLIED IN CATETHER-LIKE MEDICAL DEVICE comprising a mechanism for fastening the distal portion of the endoprosthesis, **characterized in that** it comprises a distal fastening wire (60) configured to fasten the distal portion of the endoprosthesis (17), a reinforcing tube (70) which comprises four ways (70a), (70b), (70c) and (70d) respectively and which comprises an end fastened to a command button (11), a second wire (200) which passes through the two ways (70c) and (70d) of the reinforcing tube (70) so as to form a turn and extend up to the button (11) of the reinforcing tube (70), a tether (190) which passes through one of the ways (70b) of the reinforcing tube (70) and a tether button (20) positioned in the reinforcing tube button (11), wherein the distal fastening wire (60) is secured to the tether (190) releasably and to the second wire (200).

2. MECHANISM FOR GUIDING AND/OR RELEASING AN ENDOPROSTHESIS WITH INJURED REGIONS OF A BLOOD VESSEL, APPLIED IN CATHETER-LIKE MEDICAL DEVICE according to claim 1, wherein the mechanism of releasing the distal portion of the endoprosthesis is **characterized by** the recess of said tether (190) towards the distal portion of the catheter with the distal fastening wire (60) in a non-interference condition.

3. MECHANISM FOR GUIDING AND/OR RELEASING AN ENDOPROSTHESIS WITH INJURED REGIONS OF A BLOOD VESSEL, APPLIED IN CATHETER-LIKE MEDICAL DEVICE, according to claim 1, wherein the mechanism for fastening the proximal portion of the endoprosthesis is **characterized in that** it comprises a fix multiway tube of five ways (40), four proximal fastening pins (90), a movable tube (80) and a reinforcing tube (70), wherein a wire (100) passes through this movable tube (80),, said wire being connected to the button in the grip (12) of the catheter.

4. MECHANISM FOR GUIDING AND/OR RELEASING AN ENDOPROSTHESIS WITH INJURED REGIONS OF A BLOOD VESSEL, APPLIED IN CATHETER-LIKE MEDICAL DEVICE according to claim 3, wherein the mechanism for releasing the proximal portion of the endoprosthesis is **characterized by** the movement of the button (12) which moves the wire (100) and this wire moves the small movable tube (80) which accordingly moves the four proximal fastening pins (90).

## Patentansprüche

1. Mechanismus zum Führen und/oder Lösen einer Endoprothese mit verletzten Regionen eines Blutgefäßes, der in einer katheterartigen medizinischen Vorrichtung angewandt wird, einen Mechanismus zum Befestigen des distalen Abschnitts der Endoprothese umfassend, **dadurch gekennzeichnet, dass** er einen distalen Befestigungsdraht (60) umfasst, der konfiguriert ist, um den distalen Abschnitt der Endoprothese (17) zu befestigen, ein Verstärkungsrohr (70), das jeweils vier Wege (70a), (70b), (70c) und (70d) umfasst, und das ein Ende umfasst, das an einer Befehlsschaltfläche (11) befestigt ist, einen zweiten Draht (200), der durch die beiden Wege (70c) und (70d) des Verstärkungsrohres (70) verläuft, um eine Biegung zu bilden und sich bis zu der Befehlsschaltfläche (11) des Verstärkungsrohres (70) zu erstrecken, ein Halteseil (190), das durch einen der Wege (70b) des Verstärkungsrohres (70) verläuft, und eine Halteseilschaltfläche (20), die in der Schaltfläche des Verstärkungsrohres (11) positioniert ist, wobei der distale Befestigungsdraht (60) lösbar an dem Halteseil (190) und an dem zweiten Draht (200) gesichert ist.

2. Mechanismus zum Führen und/oder Lösen einer Endoprothese mit verletzten Regionen eines Blutgefäßes, der in einer katheterartigen medizinischen Vorrichtung angewandt wird nach Anspruch 1, wobei der Mechanismus zum Lösen des distalen Abschnitts der Endoprothese durch die Aussparung des Halteseils (190) zum distalen Abschnitt des Katheters mit dem distalen Befestigungsdraht (60) in einem Zustand der Nicht-Einmischung gekennzeichnet ist.

3. Mechanismus zum Führen und/oder Lösen einer Endoprothese mit verletzten Regionen eines Blutgefäßes, der in einer katheterartigen medizinischen Vorrichtung angewandt wird nach Anspruch 1, wobei der Mechanismus zum Befestigen des proximalen Abschnitts der Endoprothese **dadurch gekennzeichnet ist, dass** er ein festes Mehrwegrohr mit fünf Wegen (40), vier proximale Befestigungsstifte (90), ein bewegliches Rohr (80) und ein Verstärkungsrohr (70) umfasst, wobei ein Draht (100) durch dieses bewegliche Rohr (80) hindurch verläuft, wobei der Draht mit der Schaltfläche in dem Griff (12) des Katheters verbunden ist.

4. Mechanismus zum Führen und/oder Lösen einer Endoprothese mit verletzten Regionen eines Blutgefäßes, der in einer katheterartigen medizinischen Vorrichtung angewandt wird nach Anspruch 3, wobei der Mechanismus zum Lösen des proximalen Abschnitts der Endoprothese durch die Bewegung der Schaltfläche (12) gekennzeichnet ist, welche den Draht (100) bewegt und dieser Draht das kleine bewegliche Rohr (80) bewegt, welches dementsprechend die vier proximalen Befestigungsstifte (90) bewegt.

## Revendications

1. Mécanisme de guidage et/ou de libération d'une endoprothèse avec des régions blessées d'un vaisseau sanguin, appliqué dans un dispositif médical de type cathéter comprenant un mécanisme de fixation de la partie distale de l'endoprothèse, **caractérisé en ce qu'**il comprend un fil de fixation distal (60) configuré pour fixer la partie distale de l'endoprothèse (17), un tube de renforcement (70) qui comprend quatre voies (70a), (70b), (70c) et (70d) respectivement et qui comprend une extrémité fixée à un bouton de commande (11), un deuxième fil (200) qui passe à travers les deux voies (70c) et (70d) du tube de renforcement (70) de manière à former une spire et s'étendre jusqu'au bouton (11) du tube de renforcement (70), une attache (190) qui passe à travers l'une des voies (70b) du tube de renforcement (70) et un bouton d'attache (20) positionné dans le bouton de tube de renforcement (11), où le fil de fixation distal (60) est fixé à l'attache (190) de manière amovible et au deuxième fil (200).

2. Mécanisme de guidage et/ou de libération d'une endoprothèse avec des régions blessées d'un vaisseau sanguin, appliqué dans un dispositif médical de type cathéter selon la revendication 1, dans lequel le mécanisme de libération de la partie distale de l'endoprothèse est **caractérisé par** l'évidement de ladite attache (190) vers la partie distale du cathéter avec le fil de fixation distal (60) dans un état de non-interférence.

3. Mécanisme de guidage et/ou de libération d'une endoprothèse avec des régions blessées d'un vaisseau sanguin, appliqué dans un dispositif médical de type cathéter selon la revendication 1, dans lequel le mécanisme de fixation de la partie proximale de l'endoprothèse est **caractérisé en ce qu'**il comprend un tube à voies multiples fixe de cinq voies (40), quatre broches de fixation proximales (90), un tube mobile (80) et un tube de renforcement (70), où un fil (100) passe à travers ce tube mobile (80), ledit fil étant relié au bouton dans la poignée (12) du cathéter.

4. Mécanisme de guidage et/ou de libération d'une endoprothèse avec des régions blessées d'un vaisseau sanguin, appliqué dans un dispositif médical de type cathéter selon la revendication 3, dans lequel le mécanisme de libération de la partie proximale de l'endoprothèse est **caractérisé par** le mouvement du bouton (12) qui déplace le fil (100) et ce fil déplace le petit tube mobile (80) qui déplace en conséquence les quatre broches de fixation proximales (90).
